(19) **Europäisches Patentamt / European Patent Office / Office européen des brevets**

(11) **EP 3 020 334 A1**

(12) # EUROPEAN PATENT APPLICATION

(43) Date of publication:
18.05.2016 Bulletin 2016/20

(51) Int Cl.:
*A61B 5/08* (2006.01)  *A61B 5/04* (2006.01)
*A61B 5/0408* (2006.01)

(21) Application number: **15153088.8**

(22) Date of filing: **29.01.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **11.11.2014 TW 103139129**

(71) Applicants:
• **Kinpo Electronics, Inc.**
**New Taipei City 22201 (TW)**
• **Cal-Comp Electronics & Communications Company Limited**
**New Taipei City 22201 (TW)**

(72) Inventors:
• **Haraikawa, Koichi**
**Shenkeng Dist., New Taipei City, 22201 (JP)**
• **Chien, Jen-Chien**
**22201 Shenkeng Dist., New Taipei City (TW)**
• **Hsu, Tsai-Chieh**
**22201 Shenkeng Dist., New Taipei City (TW)**
• **Liu, Chih-Wei**
**22201 Shenkeng Dist., New Taipei City (TW)**

(74) Representative: **2K Patentanwälte Blasberg Kewitz & Reichel**
**Partnerschaft mbB**
**Schumannstrasse 27**
**60325 Frankfurt am Main (DE)**

(54) **Measurement system of respiration-related signals and method of operating the same**

(57) A measurement system of respiration-related signals includes a plurality of electrodes (11, 12, 13), an ECG processing device (14), and an analysis device (15). The ECG processing device (14) acquires a first analog differential signal and a second analog differential signal through the electrodes and generates a Lead-I ECG signal and a Lead-II ECG signal which is orthogonal to the Lead-I ECG signal after processing the two analog differential signals. The analysis device (15) executes a trigonometric function to calculate angle change values of a cardiac axis (7) based on the acquired Lead-I ECG signal and the Lead-II ECG signal, and further generate a cardiac axis angle change waveform (82) according to the continuous angle change values. Accordingly, the cardiac axis angle change waveform (82) is suitable for the prediction of respiratory conditions of the participant because of the high positive correlation between the frequency of the cardiac axis angle change waveform (82) and the human respiratory frequency.

FIG.1

EP 3 020 334 A1

**Description**

BACKGROUND OF THE INVENTION

TECHNICAL FIELD

The technical field relates to a measurement of respiration-related signals, and more particularly, to a measurement system of respiration-related signals and a method of operating the same.

DESCRIPTION OF RELATED ART

In general, an ECG measuring instrument is used to measure ECG (electrocardiogram) signals of a participant.

Recently, a physical measurement clothing is developed for the participant to conveniently measure the ECG signals. In particular, a plurality of electrodes for signal measurement, a signal processing unit, and a signal transmission unit are jointly installed on the physical measurement clothing. Accordingly, the ECG signals can be directly and conveniently measured by the electrodes touching on specific locations of the participant's body after the participant wears the physical measurement clothing.

The physical measurement clothing is used to measure the ECG signals by the electrodes, however, the measured ECG signals cannot be directly calculated to generate the required signals, such as respiration-related signals for the participant's family, physician, or caregiver.

For the existing physical measurement clothing, additional devices or sensors are usually coordinately used to effectively measure other signals except the ECG signals. As a result, it might reduce the participant's willingness to wear the physical measurement clothing due to higher costs of the physical measurement clothing and the additional devices or sensors.

SUMMARY OF THE INVENTION

The present disclosure is directed to a measurement system of respiration-related signals and a method of operating the same to calculate the ECG signals measured by the electrodes to acquire a cardiac axis angle change waveform. Also, the cardiac axis angle change waveform is directly used to be the respiration-related signals for approximating respiratory conditions of the participant.

One of the exemplary embodiments, the measurement system of respiration-related signals includes a plurality of electrodes, an ECG processing device, and an analysis device. The ECG processing device acquires a first analog differential signal and a second analog differential signal through the electrodes and generates a Lead-I ECG signal and a Lead-II ECG signal. In particular, the Lead-I ECG signal is orthogonal to the Lead-II ECG signal.

The analysis device receives the Lead-I ECG signal and the Lead-II ECG signal, and executes a function conversion to generate an angle change value of the cardiac axis. Also, the analysis device continuously calculates the angle change values to generate a cardiac axis angle change waveform. Because the cardiac axis angle change waveform is correlated with a respiration-related signal, the cardiac axis angle change waveform can be used to be the respiration-related signal for approximating respiratory conditions of the participant.

The Lead-I ECG signal and the Lead-II ECG signal which is orthogonal to the Lead-I ECG signal are measured by different arrangements of the electrodes, and further the cardiac axis angle change waveform is generated by calculating the two ECG signals. Accordingly, the technical feature of the present disclosure is that the cardiac axis angle change waveform is suitable for the prediction of respiratory conditions of the participant because of the high positive correlation between the frequency of the cardiac axis angle change waveform and the human respiratory frequency.

In addition, it is convenient to simultaneously measure the ECG signal and respiratory conditions of the participant by wearing the physical measurement clothing and using the electrodes.

Furthermore, the respiration-related signals can be measured and calculated by the electrodes and specific algorithms instead of the additional devices or sensors, thereby significantly reducing costs of measuring the respiration-related signals.

It is to be understood that both the foregoing general description and the following detailed description are exemplary, and are intended to provide further explanation of the present disclosure as claimed. Other advantages and features of the present disclosure will be apparent from the following description, drawings and claims.

BRIEF DESCRIPTION OF DRAWINGS

The features of the present disclosure believed to be novel are set forth with particularity in the appended claims. The present disclosure itself, however, may be best understood by reference to the following detailed description of the present disclosure, which describes an exemplary embodiment of the present disclosure, taken in conjunction with the accompanying drawings, in which:

Fig. 1 is a block diagram of a measurement system according to a first embodiment of the present disclosure;
Fig. 2 is a schematic view of an electrode configuration according to the first embodiment of the present disclosure;
Fig. 3 is a flowchart of a method of measuring respiration-related signals according to the first embodiment of the present disclosure;

Fig. 4A is a schematic view of projecting an electrocardiacsignal on a first vector and a second vector according to the first embodiment of the present disclosure;

Fig. 4B is a schematic view of a cardiac axis angle according to the first embodiment of the present disclosure;

Fig. 5 is a schematic view of waveform comparison according to the first embodiment of the present disclosure;

Fig. 6 is a schematic view of an electrode configuration according to a second embodiment of the present disclosure;

Fig. 7 is a schematic view of an electrode configuration according to a third embodimentof the present disclosure; and

Fig. 8 is a schematic view of an electrode configuration according to a fourth embodiment of the present disclosure.

DETAILED DESCRIPTION

Reference will now be made to the drawing figures to describe the present disclosure in detail.

A measurement system of respiration-related signals (hereinafter referred to asa system) is used to measure respiration-related signals of a participant and to describe and recordrespiratory conditions of the participantaccording to the respiratory conditions of the participant according to the respiration-related signals.

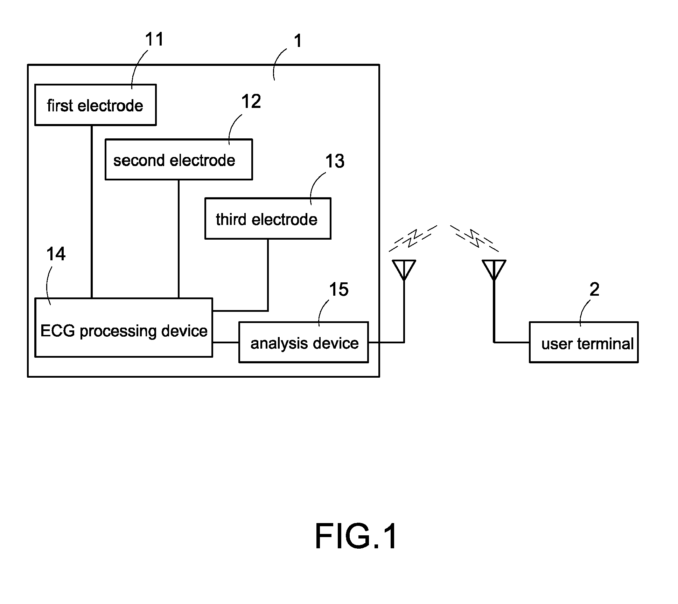

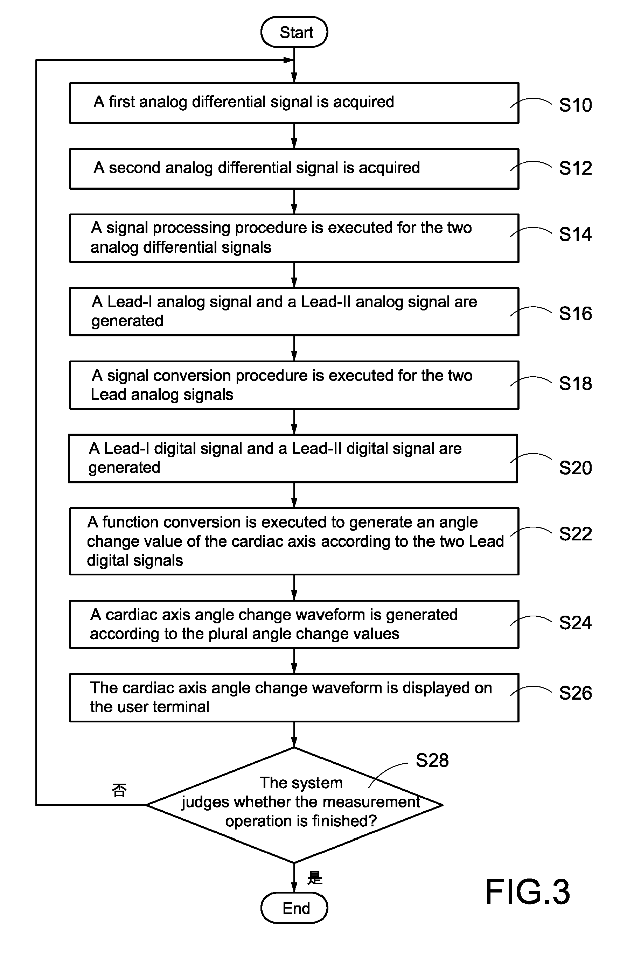

Reference is made to Fig. 1 and Fig. 3 which are a block diagram of a measurement system and a flowchart of measuring respiration-related signals according to the first embodiment of the present disclosure, respectively. As shown in Fig. 1, the system mainly includes a plurality of electrodes, an electrocardiography processing device 14 (hereinafter referred to asan ECG processing device), and an analysis device 15. In this embodiment, the ECG processing device 14 is electrically connected to the electrodes, and the analysis device 15 is connected to the ECG processing device 14 using wired or wireless manners.In a preferred embodiment of the present disclosure, the electrodes include a first electrode 11, a second electrode 12, and a third electrode 13, which are connected to the ECG processing device 14, respectively.

The electrodes 11-13 are used to directly touch the participant's body to measure electrocardiac signals and then generate ECG signals. In this embodiment, two of the three electrodes 11-13, such as the first electrode 11 and the second electrode 12 are used to acquire a first analog differential signal(S10); and two of the three electrodes 11-13, such as the second electrode 12 and the third electrode 13 are used to acquire a second analog differential signal (S12). In the present disclosure, the first analog differential signal andthe second analog differential signal are measured by a plurality of electrodes 11-13, and the analog differential signals are calculated to generate theabove-mentioned respiration-related signals. The detailed operation of the measurement system will be described hereinafter as follows.

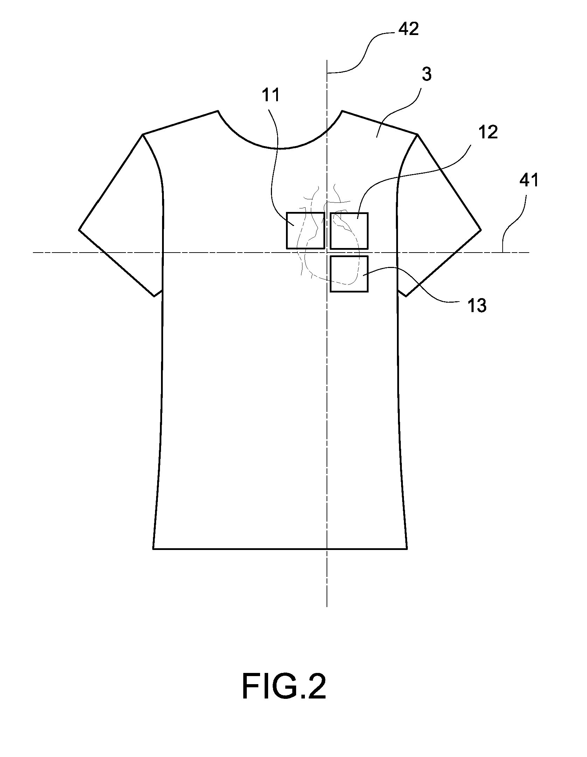

Reference is made to Fig. 2 which is a schematic view of an electrode configuration according to the first embodiment of the present disclosure. The system is preferably installed on a physical measurement clothing 3, and the participant can use the system by wearing the physical measurement clothing 3 to produce the ECG signals and calculate the respiration-related signals of the participant. Accordingly, the respiratory conditions of the participant can be viewed and recorded via the system by the participant's family, physician, orcaregiver.

As shown in Fig. 2, the electrodes 11-13 are configured on an inside surface of the physical measurement clothing 3, and the electrodes 11-13 are closely attached to the participant's bodyafter the participant wears the physical measurement clothing 3.The detailed arrangement of the electrodes 11-13 will be described hereinafter as follows.

As shown in Fig. 2, a horizontal line through the participant's heart (hereinafter referred to asacardiac horizontal line 41) and a vertical line through the participant's heart (hereinafter referred to as a cardiac vertical line 42) are illustrated. In this embodiment, the first electrode 11 is installed to firmly adhere at a second quadrant of the plain defined by the cardiac horizontal line 41 and the cardiac vertical line 42 on the physical measurement clothing 3, namely the first electrode 11 is installed to firmly adhere above the cardiac horizontal line 41 and theleft of the cardiac vertical line 42 viewed from the front of Fig. 2.The second electrode 12 is installed to firmly adhere at a first quadrant of the plain defined by the cardiac horizontal line 41 and the cardiac vertical line 42 on the physical measurement clothing 3, namely the second electrode 12 is installed to firmly adhere above the cardiac horizontal line 41 and the right of the cardiac vertical line 42 viewed from the front of Fig. 2. The third electrode 13 is installed to firmly adhere at a fourth quadrant of the plain defined by the cardiac horizontal line 41 and the cardiac vertical line 42 on the physical measurement clothing 3, namely the third electrode 13 is installed to firmly adhere below the cardiac horizontal line 41 and the right of the cardiac vertical line 42 viewed from the front of Fig. 2. However, the embodiment is only exemplified but is not intended to limit the scope of the present disclosure.

The maximum amplitude of the electrocardiac signal can be measured to acquire the completeelectrocardiographic waveform when two electrodes are vertically or horizontally installed to each other to completely cover the participant's heart.

Especially, a first vector is defined between the first electrode 11 and the second electrode 12 and a second vector is defined between the second electrode 12 and the third electrode 13, and the first vector is approximately orthogonal to the second vector. In this embodiment, an included angle between the first vector and the second vector can be 75° to 105°, and the 90-degree included angle is preferably arranged. Especially, the dominant frequency of the respiration-related signal is closer to the participant's actual respiratory frequencywhen the included angle is closer to 75°.

See Fig. 1 and Fig. 3 again, theECG processing device 14 acquires thefirst analog differential signal and the second analog differential signal via the electrodes 11-13, and then executes signal processing procedures, such as amplifying and filtering the differentialsignals (S14). Accordingly, the ECG processing device 14 can generate a Lead-I analog signal and a Lead-II analog signal using the above-mentioned signal processing procedures (S16).

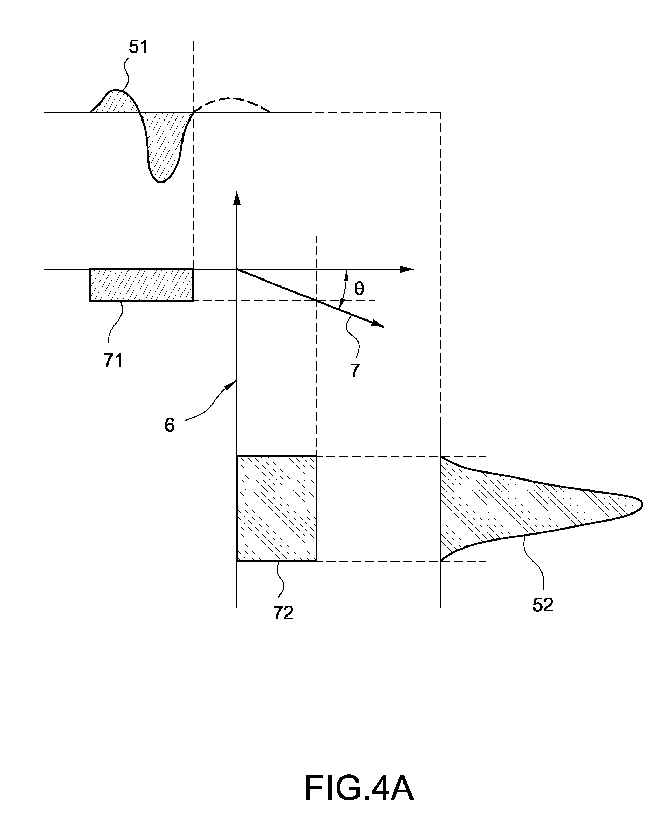

The ECG processing device 14 further executes a signal conversion procedure, such as an A/D conversion procedure of the Lead-I analog signal and the Lead-II analog signal (S18). Accordingly, the ECG processing device 14 can generate a Lead-I digital signal 51 and a Lead-II digital signal 52 shown in Fig. 4A using the A/D conversion procedure (S20).In this present disclosure, the Lead-I analog signal and the Lead-I digital signal 51 can be collectively called a Lead-I ECG signal, and the Lead-II analog signal and the Lead-II digital signal 52 can be collectively called a Lead-II ECG signal.

In this embodiment, the Lead-I digital signal 51 is generated by processing and converting the first analog differential signal, andthe Lead-II digital signal 52 is generated by processing and converting the second analog differential signal. In other words, the Lead-I digital signal 51 is measured via the first electrode 11 and the second electrode 12, and the Lead-II digital signal 52 is measured via the second electrode 12 and the third electrode 13. That is, the Lead-I digital signal 51 is derived from the first vector and the Lead-II digital signal 52 is derived from the second vector, and the an included angle between the Lead-I digital signal 51 andthe Lead-II digital signal 52 can be from 75° to 105°, and the 90-degree included angle is preferablydesigned.

After the step (S20), the ECG processing device 14 transmits the Lead-I digital signal 51 andthe Lead-II digital signal 52 to the analysis device 15 through wired or wireless manners. The analysis device 15 receives the Lead-I digital signal 51 andthe Lead-II digital signal 52 and executes a specific algorithm tocalculate an angle change valuebetween the first vector and the second vector of the cardiac axis according to the Lead-I digital signal 51 andthe Lead-II digital signal 52. More specifically, the analysis device 15 executes a function conversion to generate an angle change value of the cardiac axis according to the Lead-I digital signal 51 andthe Lead-II digital signal 52 (S22). In this embodiment, the function conversion is a trigonometric function conversion, and more particularly toatangent function conversion.

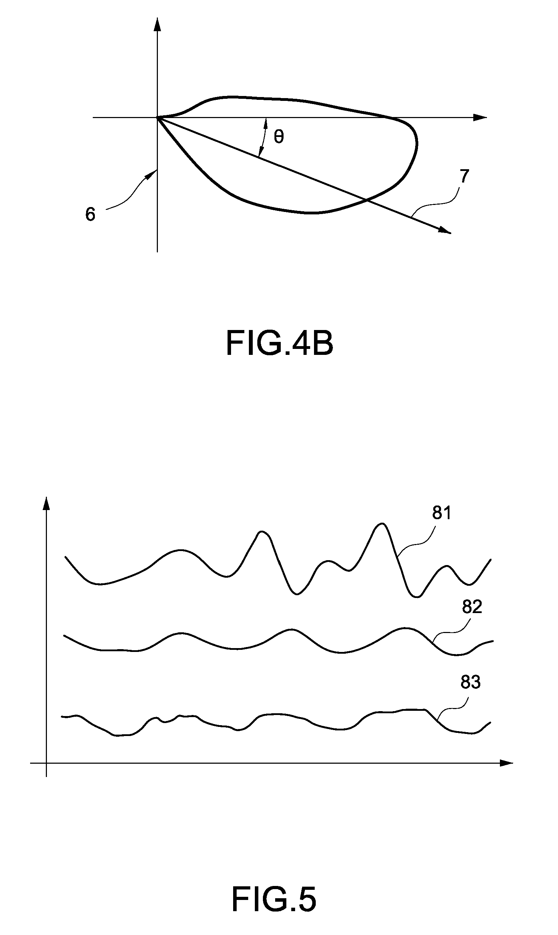

Reference is made to Fig. 4A and Fig. 4B which are schematic views of signal projections anda cardiac axis angleaccording to the first embodiment of the present disclosure, respectively.In particular, the angle change value is acquired using the trigonometric functionof a ratio between the Lead-I digital signal 51 andthe Lead-II digital signal 52.

As shown in Fig. 4A,the amplitude of the electrocardiac signal is projected on the first vector to generate the Lead-I digital signal 51, andthe Lead-I digital signal 51 is calculated by the analysis device 15 to acquire a first projection 71. Also, the amplitude of the electrocardiac signal is projected on the second vector to generate the Lead-II digital signal 52, and the Lead-II digital signal 52 is calculated by the analysis device 15 to acquire a second projection 72. In addition, the analysis device 15 executes a tangent function of the ratio between the first projection 71 and the second projection 72 to acquire the angle change value. In this embodiment, the first projection 71 and the second projection 72 are calculated by the analysis device 15 according to the amplitude area or the peak amplitude value of the Lead-I digital signal 51 and the Lead-II digital signal 52, respectively. However, the embodiments are only exemplified but are not intended to limit the scope of the presentdisclosure.

As shown in Fig. 4A, a coordinate axis 6 can be constituted by the first vector and the second vector if the first vector is orthogonal to the second vector. Accordingly, the ratio of the first projection 71 and the second projection 72 is equal to the tangent function of the included angleθ, namely:

$$tan \: \Theta = \frac{\text{first projection 71}}{\text{second projection 72}}$$

Accordingly, the calculation formula of the included angleθ is:

$$\Theta = tan^{-1} \frac{\text{first projection 71}}{\text{second projection 72}}$$

As shown in Fig. 4B, the analysis device 15 can calculate the included angle θ between the cardiac axis 7 and the coordinate axis 6 using the above-mentioned calculation formula. The included angle θ is changed when the first analog differential signal and the second analog differential signal measured by the electrodes 11-13 are changed. As a result, the angle change value of the cardiac axis 7 is generated when the included angle θ between the cardiac axis 7 and the coordinate axis 6 is changed.

See Fig. 3 again, acardiac axis angle change waveform is generated according to the plural angle change valueswhich are continuously acquired by the analysis device 15 (S24).

As shown in Fig. 1, the analysis device 15 is wirelessly connected to a user terminal 2, and the user terminal 2 provides a display device thereon. In this embodiment, the user terminal 2 can be a smart mobile device, a tablet PC, or a notebook computer, but not limited. After generating the cardiac axis angle change waveform, the analysis device 15 transmits thecardiac axis angle change waveform to the user terminal 2, andthe cardiac axis angle change waveform is displayed on the user terminal 2 (S26).In addition, a display unit can be installed in the analysis device 15 or on the physical measurement clothing 3 to directly display the cardiac axis angle change waveform from the analysis device 15 or the physical measurement clothing 3.

More specifically, the analysis device 15 generates thecardiac axis angle change waveform according to the continuous angle change values and outputs thecardiac axis angle change waveform to the display device for displaying the cardiac axis angle change waveform. In other embodiments, however, the analysis device 15 can directly output the continuous angle changevalues on the display device, thus directly displaying the continuous angle changevalues. Also, the continuous angle changevaluesconstitute the cardiac axis angle change waveform.However, these examples are for demonstration and not for limitation of the present disclosure.

After the step (S26), the system judges whether the measurement operation is finished (S28).That is, it is to judge whether the system is turned off or not, or to judge whether the electrodes 11-13 are non-contact to the participant. If the measurement operation is not finished, it is to return to the step (S10). Therefore, the electrodes 11-13 continuously measure the first analog differential signal andthe second analog differential signal, the ECG processing device 14 continuously executes the signal processing procedure and the signal conversion procedure, and the analysis device 15 continuously calculates the angle change values to generate the cardiac axis angle change waveform.

Thecardiac axis 7 would be relatively displaced to the thoracic cavity when a human breathes. Accordingly, two orthogonalLead ECG signals, such as the Lead-I digital signal 51 and the Lead-II digital signal 52 are provided to calculate the angle change value of the cardiac axis 7. In addition,the frequency of thecardiac axis angle change waveformis highly positive correlation to the human respiratory frequency.

Reference is made to Fig. 5 which is a schematic view of waveform comparison according to the first embodiment of the present disclosure.Fig. 5 illustrates an upper envelope of a single-Lead ECG measurement waveform 81, a cardiac axis angle change waveform 82, and anactual respiratory signal measurement waveform 83. The upper envelope of the single-Lead ECG measurement waveform 81 is an upper envelope of the Lead-I digital signal 51 or an upper envelope of the Lead-II digital signal 52. Thesingle-Lead ECG measurement waveform 81 is not suitable for the prediction of respiratory conditions of the participant because the upper envelope of the single-Lead ECG measurement waveform 81 is significantlydifferent from the actual respiratory signal measurement waveform 83 as shown in Fig. 5.

Obviously, the cardiac axis angle change waveform 82 is quite similar to the actual respiratory signal measurement waveform 83 as shown in Fig. 5. Accordingly, the cardiac axis angle change waveform 82 is directly used to be the respiration-related signals for approximating respiratory conditions of the participant.

Especially, the ECG signals measured by the electrodes 11-13 are not affected by therespiration-related signals. Also, one of the three electrodes 11-13 can be grounded as a reference electrode to provide a reference signal.

In the embodiment shown in Fig. 2, the electrodes 11-13 are installed on the physical measurement clothing 3, and the first electrode 11 is preferably grounded as a reference level to provide the reference signal. The preferred embodiment is shown in Fig. 2,however, the operator (user) can adjust the arrangement of the electrodes 11-13 on the physical measurement clothing 3 in accordance with the actual demand.

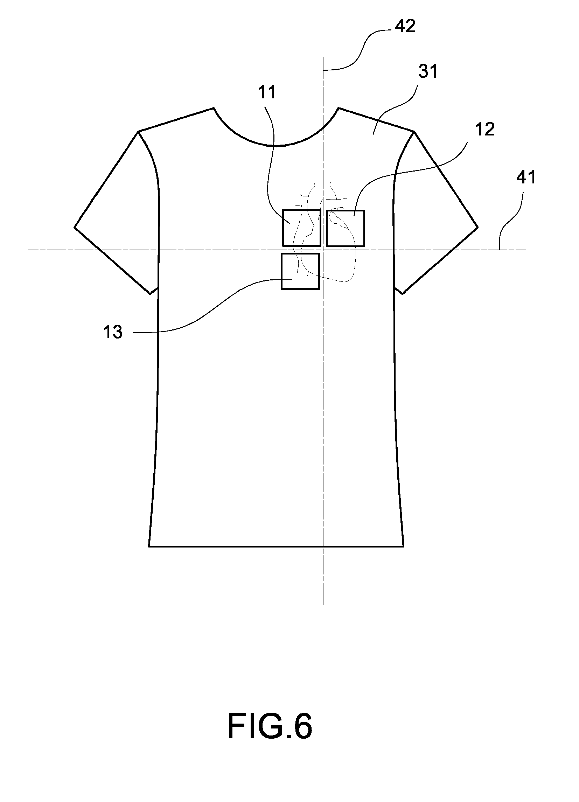

Reference is made to Fig. 6 which is a schematic view of an electrode configurationaccording to a second embodiment of the present disclosure. As shown in Fig. 6, the first electrode 11 is installed to firmlyadhere at a second quadrant of a plain defined by the cardiac horizontal line 41 and the cardiac vertical line 42 on physical measurement clothing 31, namely the first electrode 11 is installed to firmlyadhere above the cardiac horizontal line 41 and the left of thecardiac vertical line 42 viewed from the front of Fig. 6. The second electrode 12 is installed to firmly adhere at a first quadrant of the plain defined by the cardiac horizontal line 41 and the cardiac vertical line 42 on the physical measurement clothing 31, namely the second electrode 12 is installed to firmly adhere above the cardiac horizontal line 41 and the right of the cardiac vertical line 42 viewed from the front of Fig. 6. The third electrode 13 is installed to firmly adhere at a third quadrant of the plain defined by the cardiac horizontal line 41 and the cardiac vertical line 42 on the physical measurement clothing 31, namely the third electrode 13 is installed to firmly adhere below the cardiac horizontal line 41 and theleft of the cardiac vertical line 42 viewed from the front of Fig. 6.

In this embodiment, a distance between the first electrode 11 and thesecondelectrode 12 is preferably greater than a horizontal width of theparticipant's heart; and a distance between the first electrode 11 and the third electrode 13 is preferably greater than a vertical height of theparticipant's heart.

As shown in Fig. 6, the first analog differential signal is measured via the first electrode 11 and the second electrode 12 by the ECG processing device 14; and the second analog differential signal is measured via the first electrode 11 and the third electrode 13 by the ECG processing device 14. In particular, the first electrode 11 or the third electrode 13 is preferably grounded as a reference level to provide the reference signal.

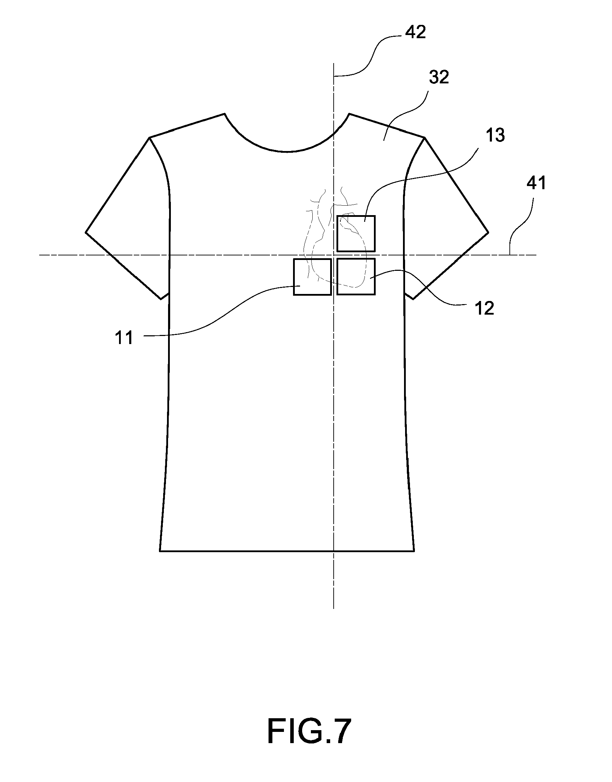

Reference is made to Fig. 7 which is a schematic view of an electrode configuration according to a third embodiment of the present disclosure. As shown in Fig. 7, the first electrode 11 is installed to firmlyadhere at a third quadrant of a plain defined by the cardiac horizontal line 41 and the cardiac vertical line 42 on a physical measurement clothing 32, namely the first electrode 11 is installed to firmlyadhere below the cardiac horizontal line 41 and the left of thecardiac vertical line 42 viewed from the front of Fig. 7. The second electrode 12 is installed to firmly adhere at a fourth quadrant of the plain defined by the cardiac horizontal line 41 and the cardiac vertical line 42 on the physical measurement clothing 32, namely the second electrode 12 is installed to firmly adhere below the cardiac horizontal line 41 and the right of the cardiac vertical line 42 viewed from the front of Fig. 7. The third electrode 13 is installed to firmly adhere at a first quadrant of the plain defined by the cardiac horizontal line 41 and the cardiac vertical line 42 on the physical measurement clothing 32, namely the third electrode 13 is installed to firmly adhere above the cardiac horizontal line 41 and the right of the cardiac vertical line 42 viewed from the front of Fig. 7.

In the embodiment, a distance between the first electrode 11 and thesecond electrode 12 is preferably greater than a horizontal width of theparticipant's heart; and a distance between thesecond electrode 12 and the third electrode 13 is preferably greater than a vertical height of the participant's heart.

As shown in Fig. 7, the first analog differential signal is measured via the first electrode 11 and the second electrode 12 by the ECG processing device 14; and the second analog differential signal is measured via the second electrode 12 and the third electrode 13 by the ECG processing device 14. In particular, the first electrode 11 is preferably grounded as a reference level to provide the reference signal.

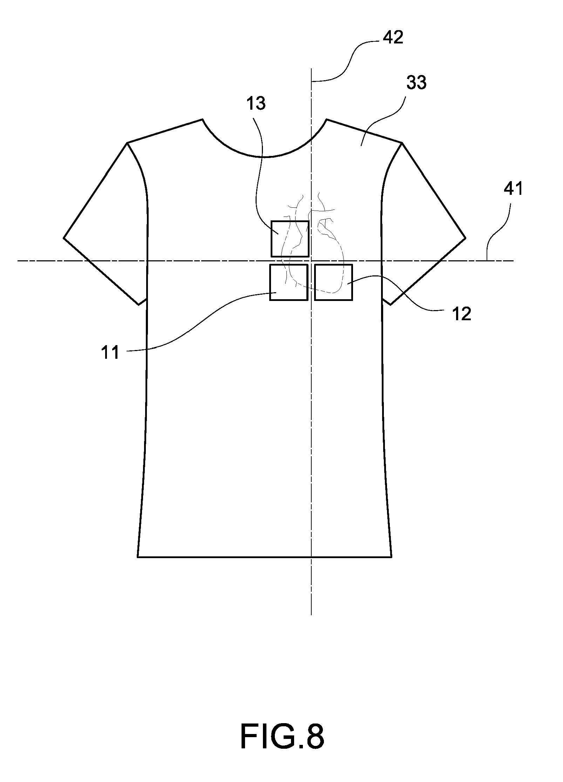

Reference is made to Fig. 8 which is a schematic view of an electrode configuration according to a fourth embodiment of the present disclosure. As shown in Fig. 8, the first electrode 11 is installed to firmlyadhere at a third quadrant of a plain defined by the cardiac horizontal line 41 and the cardiac vertical line 42 on a physical measurement clothing 33, namely the first electrode 11 is installed to firmlyadhere below the cardiac horizontal line 41 and the left of thecardiac vertical line 42 viewed from the front of Fig. 8. The second electrode 12 is installed to firmly adhere at a fourth quadrant of the plain defined by the cardiac horizontal line 41 and the cardiac vertical line 42 on the physical measurement clothing 33, namely the second electrode 12 is installed to firmly adhere below the cardiac horizontal line 41 and the right of the cardiac vertical line 42 viewed from the front of Fig. 8. The third electrode 13 is installed to firmly adhere at a second quadrant of the plain defined by the cardiac horizontal line 41 and the cardiac vertical line 42 on the physical measurement clothing 33, namely the third electrode 13 is installed to firmly adhere above the cardiac horizontal line 41 and theleft of the cardiac vertical line 42 viewed from the front of Fig. 8.

In the embodiment, a distance between the first electrode 11 and thesecond electrode 12 is preferably greater than a horizontal width of theparticipant's heart; and a distance between the first electrode 11 and the third electrode 13 is preferably greater than a vertical height of the participant's heart.

As shown in Fig. 8, the first analog differential signal is measured via the first electrode 11 and the second electrode 12 by the ECG processing device 14; and the second analog differential signal is measured via the first electrode 11 and the third electrode 13 by the ECG processing device 14. In particular, the first electrode 11 or the third electrode 13 is preferably grounded as a reference level to provide the reference signal.

In conclusion, only the three electrodes 11-13 installed on the physical measurement clothing 31-33 are used to measure the respiratory conditions of the participant, thereby improving convenience and significantly reducing costs due to the absence of external devices or sensors.

In summary there is disclosed a measurement system of respiration-related signals including a plurality of electrodes 11, 12, 13, an ECG processing device 14, and an analysis device 15. The ECG processing device 14 acquires a first analog differential signal and a second analog differential signal through the electrodes and generates a Lead-I ECG signal and a Lead-II ECG signal which is orthogonal to the Lead-I ECG signal after processing the two analog differential signals. The analysis device 15 executes a trigonometric function to calculate angle change values of a cardiac axis 7 based on the acquired Lead-I ECG signal and the Lead-II ECG signal, and further generate a cardiac axis angle change waveform (82) according to the continuous angle change values. Accordingly, the cardiac axis angle change waveform 82 is suitable for the prediction of respiratory conditions of the participant because of the high positive correlation between the frequency of the cardiac axis angle change waveform 82 and the human respiratory frequency.

**Claims**

1. A measurement system of respiration-related signals, comprising:

   a plurality of electrodes (11, 12, 13) configured to measure a first analog differential signal and a second analog differential signal of a participant;
   an ECG processing device (14) connected to the electrodes (11, 12, 13) and configured to generate a Lead-I digital signal (51) and a Lead-II digital signal (52) according to the first analog differential signal and the second analog differential signal; an included angle being between the Lead-I digital signal (51) and the Lead-II digital signal (52), andthe included angle being greater than or equal to 75° and less than or equal to 105°;

an analysis device (15) connected to the ECG processing device (14) and configured to execute a function conversion according to the Lead-I digital signal (51) and the Lead-II digital signal (52), and calculate a plurality of angle change values of a cardiac axis (7); and

a display device connected to the analysis device (15) to generatean angle change waveform (82) of a respiration-related signal according to the angle changevalues.

2. The measurement system of respiration-related signals in claim1, wherein the included angle is 90°.

3. The measurement system of respiration-related signals in claim 1 or 2,
   wherein the analysis device (15) is configured to execute the function conversion according to a ratio between the Lead-I digital signal (51) and the Lead-II digital signal (52); wherein the function conversion is executed by a tangent function conversion of the trigonometric function conversion.

4. The measurement system of respiration-related signals in claim 3, wherein the Lead-I digital signal (51) is generated by projecting an electrocardiac signal on a first vector, and the analysis device (15) is configured to calculate the Lead-I digital signal (51) to acquire a first projection (71); the Lead-II digital signal (52) is generated by projecting the electrocardiac signal on a second vector, and the analysis device (15) is configured to calculate the Lead-II digital signal (52) to acquire a second projection (72); wherein the angle changevalue is equal to:

$$\Theta = tan^{-1} \frac{\text{first projection } 71}{\text{second projection } 72}$$

when the first vector is orthogonal to the second vector.

5. The measurement system of respiration-related signals in claim 4, wherein the analysis device (15) is configured to calculate thefirst projection (71) and the second projection (72) according to an amplitude area or a peak amplitude value of the Lead-I digital signal (51) and the Lead-II digital signal (52).

6. The measurement system of respiration-related signals in claim 4 or 5, wherein the electrodes (11, 12, 13) comprises a first electrode (11), a second electrode (12), and a third electrode (13), and the electrodes (11, 12, 13) are configured on an inside surface of a physical measurement clothing (3).

7. The measurement system of respiration-related signals in claim 6, wherein the first electrode (11) is configured at a location which is above a cardiac horizontal line (41) and the right of a cardiac vertical line (42), wherein the cardiac horizontal line (41) is horizontalthrough the participant's heart andthecardiac vertical line (42) is vertically through the participant's heart; the second electrode (12) is configured at a location which is above thecardiac horizontal line (41) and the left of thecardiac vertical line (42); the third electrode (13) is configured at a location which is below the cardiac horizontal line (41) and the left of the cardiac vertical line (42); wherein the first vector is constituted by the first electrode (11) and thesecond electrode (12), and the first analog differential signal is measured by the first electrode (11) and the second electrode (12); the second vector is constituted by thesecond electrode (12) and the third electrode (13), and thesecond analog differential signal is measured by the second electrode (12) and the third electrode (13); wherein a distance between the first electrode (11) and thesecond electrode (12) is greater than a horizontal width of theparticipant's heart; and a distance between thesecond electrode (12) and the third electrode (13) is greater than a vertical height of the participant's heart.

8. The measurement system of respiration-related signals in claim 6, wherein the first electrode (11) is configured at a location which is above a cardiac horizontal line (41) and the right of a cardiac vertical line (42), wherein the cardiac horizontal line (41) is horizontal through the participant's heart andthecardiac vertical line (42) is vertically through the participant's heart; the second electrode (12) is configured at a location which is above the cardiac horizontal line (41) and the left of the cardiac vertical line (42); the third electrode (13) is configured at a location which is below the cardiac horizontal line (41) and the right of the cardiac vertical line (42); wherein the first vector is constituted by the first electrode (11) and the second electrode (12), and the first analog differential signal is measured by the first electrode (11) and the second electrode (12); the second vector is constituted by thefirst electrode (11) and the third electrode (13), and the second analog differential signal is measured by the first electrode (11) and the third electrode (13); wherein a distance between the first electrode (11) and thesecond electrode (12) is greater than a horizontal width of theparticipant's heart; and a distance between thefirst electrode (11) and the third electrode (13) is greater than a vertical height of the participant's heart.

9. The measurement system of respiration-related signals in claim 6, wherein the first electrode (11) is configured at a location which is below a cardiac horizontal line (41) and the right of a cardiac vertical line (42), wherein the cardiac horizontal line (41) is horizontal through the participant's heart and the cardiac vertical line (42) is vertically through the participant's heart; the second electrode (12) is configured at a location which is below the cardiac horizontal line (41) and the left of the cardiac vertical line (42); the third electrode (13) is configured at a location which is above the cardiac horizontal line (41) and the left of the cardiac vertical line (42); wherein the first vector is constituted by the first electrode (11) and the second electrode (12), and the first analog differential signal is measured by the first electrode (11) and the second electrode (12); the second vector is constituted by the second electrode (12) and the third electrode (13), and the second analog differential signal is measured by the second electrode (12) and the third electrode (13); wherein a distance between the first electrode (11) and the second electrode (12) is greater than a horizontal width of the participant's heart; and a distance between the second electrode (12) and the third electrode (13) is greater than a vertical height of the participant's heart.

10. The measurement system of respiration-related signals in claim 6, wherein the first electrode (11) is configured at a location which is below a cardiac horizontal line (41) and the right of a cardiac vertical line (42), wherein the cardiac horizontal line (41) is horizontal through the participant's heart and the cardiac vertical line (42) is vertically through the participant's heart; the second electrode (12) is configured at a location which is below the cardiac horizontal line (41) and the left of the cardiac vertical line (42); the third electrode (13) is configured at a location which is above the cardiac horizontal line (41) and the right of the cardiac vertical line (42); wherein the first vector is constituted by the first electrode (11) and the second electrode (12), and the first analog differential signal is measured by the first electrode (11) and the second electrode (12); the second vector is constituted by the first electrode (11) and the third electrode (13), and the second analog differential signal is measured by the first electrode (11) and the third electrode (13); wherein a distance between the first electrode (11) and the second electrode (12) is greater than a horizontal width of the participant's heart; and a distance between the first electrode (11) and the third electrode (13) is greater than a vertical height of the participant's heart.

11. A method of measuring respiration-related signals, applied to a measurement system (1) of a respiration-related signal, the measurement system (1) comprising a plurality of electrodes (11, 12, 13) installed to firmly adhere on a participant's heart, the method comprising:

(a) acquiring a first analog differential signal and a second analog differential signal;
(b) generating a Lead-I digital signal (51) and a Lead-II digital signal (52) according to the first analog differential signal and the second analog differential signal; an included angle being between the Lead-I digital signal (51) and the Lead-II digital signal (52), and the included angle being greater than or equal to 75° and less than or equal to 105°; and
(c) executing a function conversion according to Lead-I digital signal (51) and the Lead-II digital signal (52), and calculating a plurality of angle change values of a cardiac axis (7).

12. The method of measuring respiration-related signals in claim 11, wherein the step (b) comprises:

(b1) generating a Lead-I analog signal and a Lead-II analog signal by executing a signal processing procedure to the first analog differential signal and the second analog differential signal; and
(b2) generating the Lead-I digital signal (51) and the Lead-II digital signal (52) by executing a signal conversion procedure to the Lead-I analog signal and the Lead-II analog signal.

13. The method of measuring respiration-related signals in claim 11 or 12, wherein the included angle is 90°.

14. The method of measuring respiration-related signals in claim 11 or 13, wherein in the step (c), the function conversion is executed according to a ratio between the Lead-I digital signal (51) and the Lead-II digital signal (52); wherein the function conversion is executed by a tangent function conversion of the trigonometric function conversion.

15. The method of measuring respiration-related signals in claim 14, wherein in the step (c), the Lead-I digital signal (51) is generated by projecting an electrocardiac signal on a first vector, and the Lead-I digital signal (51) is calculated to acquire a first projection (71); the Lead-II digital signal (52) is generated by projecting the electrocardiac signal on a second vector, and the Lead-II digital signal (52) is calculated to acquire a second projection (72); wherein the angle change value is equal to:

$$\Theta = tan^{-1} \frac{\text{first projection 71}}{\text{second projection 72}}$$

when the first vector is orthogonal to the second vector.

16. The method of measuring respiration-related signals in claim 15, wherein in the step (c), the first projection (71) and the second projection (72) are calculated according to an amplitude area or a peak amplitude value of the Lead-I digital signal (51) and the Lead-II digital signal (52).

FIG.1

FIG.2

Start

A first analog differential signal is acquired — S10

A second analog differential signal is acquired — S12

A signal processing procedure is executed for the two analog differential signals — S14

A Lead-I analog signal and a Lead-II analog signal are generated — S16

A signal conversion procedure is executed for the two Lead analog signals — S18

A Lead-I digital signal and a Lead-II digital signal are generated — S20

A function conversion is executed to generate an angle change value of the cardiac axis according to the two Lead digital signals — S22

A cardiac axis angle change waveform is generated according to the plural angle change values — S24

The cardiac axis angle change waveform is displayed on the user terminal — S26

S28

否 — The system judges whether the measurement operation is finished?

是

End

FIG.3

FIG.4A

FIG.4B

FIG.5

FIG.6

FIG.7

FIG.8

Europäisches
Patentamt

European
Patent Office

Office européen
des brevets

# EUROPEAN SEARCH REPORT

Application Number

EP 15 15 3088

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 415 174 B1 (BEHBEHANI KHOSROW [US] ET AL) 2 July 2002 (2002-07-02) <br> * column 2, lines 15-43 * <br> * column 5, lines 57-67 * <br> * column 6, lines 1-29 * <br> * column 7, line 45 - column 8, line 36 * <br> * column 9, lines 29-60 * <br> * claims; figures * <br> ----- | 1-16 | INV. <br> A61B5/08 <br> A61B5/04 <br> A61B5/0408 |
| X | WO 2009/043087 A1 (COMPUMEDICS MEDICAL INNOVATION [AU]; NILSEN KRIS [AU]; ZILBERG EUGENE) 9 April 2009 (2009-04-09) <br> * page 10, line 21 - page 11, line 25 * <br> * claims * <br> ----- | 1-16 | |
| A | US 5 694 939 A (COWINGS PATRICIA S [US]) 9 December 1997 (1997-12-09) <br> * column 6, line 66 - column 7, line 12 * <br> * figure 2 * <br> ----- | 1-16 | |
| A | WO 2006/095279 A1 (PHILIPS INTELLECTUAL PROPERTY [DE]; KONINKL PHILIPS ELECTRONICS NV [NL] 14 September 2006 (2006-09-14) <br> * the whole document * <br> ----- | 1-16 | TECHNICAL FIELDS SEARCHED (IPC) <br><br> A61B |
| A | US 2014/163334 A1 (VOLPE SHANE S [US] ET AL) 12 June 2014 (2014-06-12) <br> * paragraphs [0003], [0029] * <br> * figure 1 * <br> ----- | 1-16 | |
| A | WO 2011/109515 A2 (VOLKER MONICA ANN [US]) 9 September 2011 (2011-09-09) <br> * the whole document * <br> ----- | 1-16 | |

-/--

The present search report has been drawn up for all claims

| Place of search | Date of completion of the search | Examiner |
|---|---|---|
| The Hague | 8 April 2015 | Bataille, Frédéric |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................
& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 15 15 3088

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | MOODY G B ET AL: "Derivation of respiratory signals from multi-lead ECGs", PROCEEDINGS OF THE COMPUTERS IN CARDIOLOGY MEETING. LINKOPING, SWEDEN, SEPT. 8 - 11, 1985; [PROCEEDINGS OF THE MEETING ON COMPUTERS IN CARDIOLOGY], WASHINGTON, IEEE COMP. SOC. PRESS, US, vol. MEETING 12, 8 September 1985 (1985-09-08), pages 113-116, XP002962678, * the whole document * | | |

-----

TECHNICAL FIELDS
SEARCHED      (IPC)

The present search report has been drawn up for all claims

| Place of search | Date of completion of the search | Examiner |
|---|---|---|
| The Hague | 8 April 2015 | Bataille, Frédéric |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

    .................................................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 15 15 3088

This annex lists the patent family members relating to the patent documents cited in the above-mentioned European search report.
The members are as contained in the European Patent Office EDP file on
The European Patent Office is in no way liable for these particulars which are merely given for the purpose of information.

08-04-2015

| Patent document cited in search report | | Publication date | Patent family member(s) | | Publication date |
|---|---|---|---|---|---|
| US 6415174 | B1 | 02-07-2002 | NONE | | |
| WO 2009043087 | A1 | 09-04-2009 | AU | 2008307127 A1 | 09-04-2009 |
| | | | CA | 2697631 A1 | 09-04-2009 |
| | | | CN | 101815465 A | 25-08-2010 |
| | | | EP | 2203115 A1 | 07-07-2010 |
| | | | JP | 5303802 B2 | 02-10-2013 |
| | | | JP | 2010540124 A | 24-12-2010 |
| | | | KR | 20100045521 A | 03-05-2010 |
| | | | US | 2010217133 A1 | 26-08-2010 |
| | | | WO | 2009043087 A1 | 09-04-2009 |
| US 5694939 | A | 09-12-1997 | NONE | | |
| WO 2006095279 | A1 | 14-09-2006 | EP | 1871225 A1 | 02-01-2008 |
| | | | US | 2008208029 A1 | 28-08-2008 |
| | | | WO | 2006095279 A1 | 14-09-2006 |
| US 2014163334 | A1 | 12-06-2014 | DE | 102008020562 A1 | 02-01-2009 |
| | | | FR | 2917298 A1 | 19-12-2008 |
| | | | JP | 5478034 B2 | 23-04-2014 |
| | | | JP | 2008307382 A | 25-12-2008 |
| | | | JP | 2014121629 A | 03-07-2014 |
| | | | US | 2008312709 A1 | 18-12-2008 |
| | | | US | 2010312297 A1 | 09-12-2010 |
| | | | US | 2014163334 A1 | 12-06-2014 |
| WO 2011109515 | A2 | 09-09-2011 | US | 2013053674 A1 | 28-02-2013 |
| | | | WO | 2011109515 A2 | 09-09-2011 |

EPO FORM P0459

For more details about this annex : see Official Journal of the European Patent Office, No. 12/82